# EUROPEAN PATENT APPLICATION

(11) **EP 3 702 988 A1**
(43) Date of publication of application: **02.09.2020**
(21) Application number: 19160084.0
(22) Date of filing: 28.02.2019
(51) Int. Cl.: G06Q 10/10, G16H 10/60

(54) **COMPUTER-IMPLEMENTED SYSTEM AND METHOD FOR SCHEDULING HEALTHCARE APPOINTMENTS**

(71) Applicant: Caldr, 9240 Zele (BE)
(72) Inventor: Moerman, Jochen, 9040 Gent (BE)
(74) Representative: IP HILLS NV

(57) **Abstract**

A computer-implemented system (100) for scheduling a new healthcare appointment configured for:
- registering respective times of click events in electronic patient files;
- determining actual start times and end times of past healthcare appointments from the click events;
- maintaining an average duration for a healthcare appointment per care provider, per patient and per consultation type from the start times and said end times;
- receiving a scheduling request for the new healthcare appointment, the request comprising a patient identification, a care provider identification, and a consultation type identification; and
- determining an average duration for the new healthcare appointment based on the patient identification, the care provider identification and the consultation type identification; and
- selecting in an appointment schedule an available timeslot of the average duration for the new healthcare appointment.

## Description

### Field of the Invention

The present invention generally relates to scheduling healthcare appointments, like for example the scheduling of a healthcare consultation by a patient with a care provider, for instance a physician, a dentist, a physiotherapist, etc., or with a healthcare cabinet or other organisation associating plural care providers.

### Background of the Invention

When scheduling an appointment for healthcare consultation or assistance with a care provider, a patient is often confronted with a long waiting list. In case of a scheduled appointment, the patient often still has to spend a long waiting time in the waiting room of the care provider. Care providers on the other hand are confronted with no-show behaviour of patients that schedule an appointment at a certain date/time but do not cancel the appointment when the consultation with the care provider is no longer needed. As a consequence thereof, care providers suffer loss of time and income due to patients not showing up at healthcare appointments while other patients wait unnecessarily long for their healthcare consultations, leading to frustration with care providers and patients.

Existing online applications for scheduling healthcare appointments that typically provide the functionality of a calendar wherein patients can book available, fixed length timeslots for an appointment, are not optimal in terms of patient interaction and patient engagement. The fixed length timeslots that can be booked therein do not consider the type of consultation, for instance the urgency and/or the type of treatment, nor do they consider the type of patient. In case of delay/advance in view of the daily schedule of a care provider, either the care provider or his patients will lose time. No-show behaviour by patients remains an unsolved, growing problem and late cancelled appointments require an assistant of the care provider to manually contact other patients to reschedule their appointments in order to avoid the care provider is losing time and income.

United States Patent Application US 2010/0185465 A1 entitled "Electronic Appointment Scheduling for Medical Resources" describes a computer-implemented system for scheduling patient appointments. The system is adapted for receiving a patient identifier and a selected reason for the requested medical appointment. The system thereto consults an electronic medical record associated with the patient identifier and generates therefrom a patient-specific list of available reasons for a medical appointment for the patient where a selection can be made from. The patient shall use his patient interface terminal to select a reason for the appointment and a timeslot will be scheduled that is based on the reason of the appointment, thereby increasing efficiency for the care provider. The system known from US 2010/0185465 thus can tailor the amount of time necessary for an appointment to the reason for the appointment and filters medical services available to the patient based on the patient identity and the patient's electronic medical record.

The system known from US 2010/0185465 primarily aims at efficiently controlling the use of medical resources and costs. Scheduling medical providers, equipment, laboratory services or other medical resources by a patient is made conditional to medical services being available for that patient. The system however does not address the problem of no-show behaviour of patients, neither does it consider that the time required for the same medical service may depend on the care provider and/or on the patient receiving the service. The system known from US 2010/0185465 does not increase the efficiency for patients, neither does it enhance the patient's engagement to appointments, therapy and/or use of medication.

United States Patent Application US 2015/0242819 A1 entitled "Systems and Methods for Improving Scheduling Inefficiencies Using Predictive Models" recognizes the risk of no-shows or late cancellations by patients and therefore describes a system that predicts no-show behaviour of a patient based on historical data, i.e. information indicating whether a patient showed up to past appointments, cancelled in advance, or did not show-up. A predictive model based on logistic regression, support vector machines or neural networks predicts that a new appointment in a particular timeslot will probably be a no-show or late cancellation. The scheduling system decides to double-book such timeslot in order to reduce the risk of loss of time and income for a care provider. The scheduling system known from US 2015/0242819 also transmits messages to the appointment-holder and/or schedules telephone calls to the appointment-holder in view of the no-show risk.

The system known from US 2015/0242819 optimizes the time and revenue income for care providers, but fails to recognize the needs of patients that want to see the waiting time for their appointment reduced, that want to schedule timeslots that fit the medical services they require, that prefer to stay aware of delay/advance of a care provider in his daily schedule such that time otherwise spent in the waiting room can be used more efficiently, like for instance shopping, household tasks, or professional duties.

United States Patent Application US 2012/0053963 A1 entitled "System for Dynamically Scheduling Medical Facility Appointments" describes a system that dynamically reschedules medical appointments in the event a patient plans to be late or cancels an appointment, and that dynamically reschedules medical appointments in the event a medical service provider unexpectedly becomes unavailable, for instance as a result of an emergency he has to attend to.

The system known from US 2012/0053963 however works with fixed timeslots as a result of which it is not very efficient. Delays and advances in the daily schedule of a care provider are inherent to such system with fixed length timeslots, hence negatively impacting the efficient use of time for both care providers and patients. The system known from US 2012/0053963 also does not predict no-show behaviour by patients. Instead of anticipating, the system is reactive on no-shows and late cancellations and therefore expectations that it can improve the efficiency for care providers are possibly overestimated. Further, the system of US 2012/0053963 fails to enhance patient engagement as no measures are taken to reduce no-shows or late cancellations and/or to follow-up on medical therapy.

United States Patent Application US 2009/0177489 A1 entitled "Systems and Methods for Patient Scheduling and Record Handling" describes linking the management of electronic patient records to the scheduling of medical appointments. In order to enhance the productivity in the medical economy, patient-specific data indicative for the patient's cancellation history and the patient's payment history enhance the electronic patient record such that the appointment scheduling process can be optimized. Automated real-time alerts are generated for the patient and healthcare provider in view of the patient's payment record and cancellation history to mitigate costs and loss of time of the healthcare provider.

In US 2009/0177489 no measures are taken to adapt timeslots for medical appointments to the context of the appointment, e.g. the reason, the care provider, the patient. US 2009/0177489 also fails to enhance patient engagement towards medical appointments and/or prescribed therapy.

Summarizing, there is a general need for improved computer-implemented systems and methods for scheduling healthcare appointments that further optimize the time management of care providers while at the same time enhancing patient engagement by reducing no-show behaviour, late cancellations, and waiting times for patients as well as by increasing therapy engagement.

### Summary of the Invention

The above defined object is realized as one or several of the above identified shortcomings of existing systems are resolved by the computer-implemented system for scheduling a new healthcare appointment defined by claim 1, comprising:
- a first computer system configured for:
   - registering respective times of click events in electronic patient files, an electronic patient file being a digital record comprising a patient identifier of a patient and health data concerning the patient;
   - determining actual start times and end times of past healthcare appointments from the click events; and
   - maintaining an average duration for a healthcare appointment per care provider, per patient and per consultation type from the start times and the end times;
- a user interface terminal configured for:
   - receiving a scheduling request for the new healthcare appointment, the request comprising a patient identification, a care provider identification, and a consultation type identification; and
- a second computer system configured for:
   - receiving the patient identification, the care provider identification and the consultation type identification;
   - receiving from the first computer system an average duration based on the patient identification, the care provider identification and the consultation type identification; and
   - selecting in an appointment schedule an available timeslot of the average duration for the new healthcare appointment.

Thus, the invention concerns a computer-implemented, virtual back-office assistant for a care provider or an association of care providers that dynamically automates the scheduling of healthcare appointments. A first computer system connects with an electronic patient file database and monitors click events in electronic patient files, like for instance the opening of an electronic patient file by a care provider or his assistant, the closing of an electronic payment file by a care provider or his assistant, etc. The times of such click events are registered and enable the first computer system to estimate the actual start time of a healthcare appointment, e.g. corresponding to the time whereon an electronic patient file is opened, or the time of a first click in an electronic patient file after it has been opened, or the time a dedicated button displayed in or near the electronic patient file is clicked, etc. The times of such click events also enable the first computer system to estimate the end time of a healthcare appointment, e.g. corresponding to the time whereon the electronic patient file is closed, or the time of a last click in an electronic patient file before it is closed, or the time a dedicated button displayed in or near the electronic patient file is clicked, the time an electronic payment related to the electronic patient file is confirmed, etc. In addition to clicks, the first computer system in embodiments of the system according to the invention also may monitor other electronically detectable events or interactive behaviour in electronic patient files such as scrolling, the entry or manipulation of data, movements of the cursor or mouse pointer, etc., by the care provider or his assistant. The monitoring of such events or behaviour shall allow the first computer system to more accurately determine the start time and end time of a healthcare appointment.

The electronic patient file in the context of the present invention must be interpreted broadly to cover any record that comprises at least a patient identification and health data related to the patient, such as age, gender, allergies, medical history, past medical treatment, medication or other therapy followed, etc. The electronic patient files may be named Electronic Health Record (EHR), Electronic Medical Record (EMR), Electronic Patient Record (EPR), Electronic Patient Datafile (EPD), etc., and may be stored in an electronic patient file database external or internal to the computer-implemented system according to the invention. The electronic patient file database may for instance be managed by a governmental organisation, by a healthcare organisation like a hospital or healthcare cabinet, by a healthcare fund, by a single care provider, etc.

Essential to the present invention is that automated monitoring of clicks in electronic patient files enables a first computer system to estimate the effective length in time of healthcare appointments, and to dynamically determine therefrom the average duration of healthcare appointments per consultation type, per care provider and per patient. Indeed, the duration of a healthcare appointment depends on the consultation type, i.e. the urgency, reason and/or type of treatment, but the duration of a healthcare appointment shall also vary depending on the care provider, i.e. the experience of the care provider and the fact that a certain care provider spends more time than his colleague for the same or similar diagnostic tests, and the duration of a healthcare appointment shall also vary depending on the patient, i.e. certain patients are more difficult to diagnose than others, certain patients spend more time to small talk with the care provider, etc. The first computer system that forms part of the system according to the invention dynamically learns over time the average duration of a healthcare appointment per consultation type, per care provider and gets this intelligence from monitoring clicks in electronic patient files that are always used by care providers during an appointment. It is noticed that the click events may come from the care provider, the care provider's assistant or other healthcare personnel like for instance a receptionist in a healthcare cabinet, etc. It is also noticed that averaging in the context of the present invention must not be interpreted restrictively to cover only the mathematical average but encompasses alternative averaging techniques like determining the median value, removing outliers, weighted averaging such that for instance higher weights are attributed to more recent past healthcare appointments, etc.

Whenever a patient schedules a new healthcare appointment, the intelligence gathered by the first computer system can be exploited by a second computer system to determine the time interval required for such new healthcare appointment. Identification of the patient, care provider and consultation type in the request for the new appointment is sufficient for the second computer system to obtain from the first computer system the corresponding average duration. The second computer system further connects with an appointment schedule, for example an electronic calendar of the care provider identified in the request for the new healthcare appointment, and searches therein one or several available timeslots with length equal to or exceeding the average duration. These timeslots are proposed to the patient who makes a choice as a result of which the new healthcare appointment is confirmed and the corresponding timeslot with length equal to the average duration is booked in the care provider's schedule. The length of the timeslot reserved for the new healthcare appointment consequently is tuned dynamically to the consultation type, the care provider and the patient. The risk that the healthcare appointment will take substantially less time as a result of which the care provider would lose time and income, and the risk that the healthcare appointment will take substantially more time as a result of which other patients would spend more time waiting and the care provider has to work overtime, are minimized to the satisfaction of both the care provider and his patients. Since waiting times are minimized, patients will be less motivated to no-shows or late cancellations, hence increasing patient engagement towards scheduled appointments and further reducing the loss of time and income for care providers. The second computer system may suggest timeslots in the schedule of an alternate care provider, other than the one identified in the request for the new healthcare appointment, when for instance no timeslot of appropriate duration is available within a predetermined time period of one or several days in the schedule of the care provider identified in the request. In such case, the second computer system obviously has to consult the first computer system to obtain therefrom the average duration for a healthcare appointment of the same type, for the same patient, but with a different care provider. As explained here above, this average duration may differ and therefore also the length of the available timeslot that will be searched in the schedule of the alternate care provider. It is noticed that the length of the available timeslot searched in the schedule of an alternate care provider may also account for allowable delay of that alternate care provider. In case the maximum allowable delay of the alternate care provider is set at 15 minutes, an available timeslot of 20 minutes in the schedule of the alternate care provider may for instance be booked for a healthcare appointment that is estimated to have a duration of 30 minutes. The remaining appointments in the alternate care provider's schedule for that day will have a theoretic delay of 10 minutes in such case which is still below the maximum allowable delay set by the alternate care provider.

It is further noticed that although the system according to the invention is specified to comprise a first computer system and a second computer system, any person skilled in the art of computer-implemented systems for healthcare applications shall appreciate that the first computer system and second computer system in embodiments of the present invention may be integrated to form a single processing system, or in alternate embodiments may be distributed over more than two processing systems providing the functionality of the first and second computer system.

In embodiments of the computer-implemented system for scheduling a new healthcare appointment according to the invention, as defined by claim 2, the first computer system is further configured for:
- maintaining a no-show score per patient and increasing the no-show score whenever no electronic patient file is opened and/or no patient identification card is scanned for a scheduled healthcare appointment of the patient, and/or the electronic patient file is opened only during a time interval shorter than a certain time limit, and/or a new medical appointment is multiple times rescheduled by the patient, and/or similar new medical appointments are scheduled by the patient with multiple care providers, and/or the response delay to a request sent to the patient exceeds a certain threshold.

Indeed, monitoring click events in electronic patient files enables improved embodiments of the system according to the present invention to detect no-show behaviour of a patient, for instance when the electronic patient file is not opened at all at the day an appointment was scheduled (or is opened only during a very short time interval of for instance a few seconds) or when no patient ID is scanned at the time the healthcare appointment was scheduled. Other indicators of no-show behaviour are for instance the scheduling of multiple appointments with multiple care providers for a same issue, slow responsiveness to requests sent to a patient (for example a long delay is noticed between receipt of a message and the actual response to a message, in particular when the response is short), repeatedly cancelling and re-scheduling a healthcare appointment, etc. Embodiments of the invention may implement monitoring a subset of one or more of these indicators in order to maintain the patient's no-show score. This enables to maintain and update a no-show score for each patient indicative for the engagement of a patient. A high no-show score, being a no-show score that exceeds a certain predetermined no-show threshold value, may be used to install measures that should increase the patient's engagement. These measures may for instance entail automated interaction fuelling intrinsic motivation in respect of the patient, sending reminders or alerts to such patient for future healthcare appointments, using different channels for sending reminders/alerts, increasing the reminder/alert frequency, requesting confirmation for future healthcare appointments, allowing double bookings in timeslots reserved for future healthcare appointments (for instance in absence of confirmation), requesting upfront payment of a no-show fee for future healthcare appointments, automated therapy follow-up, etc. This way, no-show behaviour will reduce over time and patient engagement will increase to the benefit of the care provider's efficiency.

In embodiments of the computer-implemented system for scheduling a new healthcare appointment according to the invention, as defined by claim 3, the second computer system is further configured for:
- determining if an automatic reminder is sent to a patient for a future healthcare appointment based on said no-show score of said patient.

Indeed, a straightforward measure to implement consists of automated interaction, sending automatic reminders/alerts to a patient for an upcoming healthcare appointment when the no-show risk of this patient exceeds a certain no-show threshold level. The amount and/or the frequency of such reminder or alert messages may increase with increasing no-show scores.

In further embodiments of the computer-implemented system for scheduling a new healthcare appointment according to the invention, as defined by claim 4, the second computer system is further configured for:
- selecting a communication channel for the reminder based on the no-show score of the patient.

Indeed, depending on the no-show score, different channels may be used to send the reminders/alerts. An e-mail message may for instance be sent to patients whose no-show score exceeds a first no-show threshold, an sms message may be sent to patients whose no-show score exceeds a second no-show threshold higher than the first no-show threshold, a telephone call may be made to patients whose no-show score exceeds a third no-show threshold higher than the second no-show threshold and the care provider or his assistant will be prompted to make such telephone call, etc. Alternate communication channels, for example Instant Messaging like WhatsApp, Hangout, ... or other applications like for instance a proprietary application integrated in the system according to the invention may be used, plural channels may be combined to remind the patient of the upcoming scheduled healthcare appointment. A cascade of non-intrusive notification (like for instance Instant Messaging) up to intrusive notification (like for instance a telephone call) may be implemented, depending on the no-show score of the patient. A patient with a high no-show score that is responsive to a WhatsApp message consequently still may be alerted for an upcoming healthcare appointment through other channels.

In embodiments of the computer-implemented system for scheduling a new healthcare appointment according to the invention, as defined by claim 5, the second computer system is further configured for:
- following-up on a therapy or medication through automated interrogation of a patient after a healthcare appointment based on the no-show score of the patient.

A high no-show score for a patient is indicative for low engagement by the patient, not only towards future scheduled appointments but also towards a therapy recommended by a care provider as a result of a consultation. The therapy may for instance be daily medication, periodic visits to a physiotherapist, following a diet, etc. The system according to the present invention therefore may automatically notify a patient (for instance at points in time where medication must be taken), or interact with a patient through automated interrogation of the patient with respect to the prescribed therapy, evolution as a result of medication, possible side-effects of medication, etc. In addition to increasing the patient's therapy engagement, this also enables to monitor effectiveness and/or side-effects of a therapy.

In embodiments of the computer-implemented system for scheduling a new healthcare appointment according to the present invention, as defined by claim 6, the second computer system is further configured for:
- monitoring in the appointment schedule progression of scheduled appointments per care provider based on said start times and end times, and determining delay/advance of the care provider in view of his daily schedule;
- automatically notifying patients of one or plural upcoming appointments with the care provider on the delay/advance if the delay/advance exceeds a certain threshold; and
- registering an adequate delay for notifying patients of such delay/advance.

Embodiments of the system according to the present invention thus preferably real-time monitor progression of a care provider in his daily schedule. As soon as a delay is detected that exceeds a predetermined threshold, for instance 30 minutes, the next few patients in line may be notified automatically of such delay. Notifying patients of delay/advance in the daily schedule of a care provider shall preferably respect an adequate delay, for instance 1 hour, enabling the patient to anticipate for the delay/advance. This way, unnecessary waiting and frustration by such patients in the waiting room of the care provider are avoided when the care provider is for instance called for an emergency. The patients notified of such delay may usefully spend the delay period, for instance for household tasks, professional tasks, groceries or shopping, and appear later at the care provider's cabinet. Alternatively, the patients notified of such delay may be given the possibility to cancel their appointment and/or re-schedule their appointment, hence enabling the care provider to catch up with his daily schedule. Also, when an advance in the daily scheme is detected that exceeds a certain threshold, for instance 30 minutes, the next few patients in line may be notified automatically of such advance, again respecting an adequate delay enabling the patient to anticipate. This way, patients are given the chance to come earlier to the care provider's cabinet for their consultation, reducing waiting time for the care provider and enabling the care provider to end his working day earlier or to allow one or several additional patients to schedule appointments at the end of the day. Dynamic real-time monitoring of advance/delay in a care provider's daily schedule dynamically minimizes both the care provider's waiting time and the patients' waiting time throughout the day.

In embodiments of the computer-implemented system for scheduling a new healthcare appointment according to the invention, as defined by claim 7, the second computer system is further configured for:
- automatically re-scheduling all appointments scheduled for a day with a care provider if the care provider indicates absence for the day; and
- generating re-schedule proposals for patients of the appointments, each re-schedule proposal comprising a respective new date and respective new timeslot of appropriate duration, available in the appointment schedule for the care provider.

Thus, advanced embodiments of the system according to the present invention automatically re-schedule appointments with a care provider when the care provider indicates absence for a certain period, for instance because the care provider is ill, attends a conference, follows a training, is called for an emergency that might take several hours, etc. All patients that have a healthcare appointment scheduled within the period where the care provider indicates absence, are contacted automatically by the system with a notification that their appointment is cancelled and further receive a proposed new date/time for a re-scheduled appointment with the care provider. The automatic re-scheduling saves time and manual intervention by the care provider or his assistant to re-schedule the appointments, further increasing the efficiency of the care provider.

In embodiments of the computer-implemented system for scheduling a new healthcare appointment according to the invention, as defined by claim 8, the computer system is further configured for:
- determining from the click events an inter-consultation time between closing of an electronic patient file and opening of a following electronic patient file; and
- maintaining an average inter-consultation time per care provider, per patient, and per consultation type therefrom,
and wherein the second computer system is further configured for:
- receiving from the first computer system an average inter-consultation time based on the patient identification, the care provider identification and the consultation type identification; and
- adding the inter-consultation time interval to the average duration when selecting in an appointment schedule an available timeslot for the new healthcare appointment.

Thus, monitoring click events in electronic patient files also enables the system according to the invention to determine the average inter-consultation time per care provider, per patient and per consultation type. The inter-consultation time may be used by the care provider for small talk with the patient, to clean his cabinet, to perform some administrative tasks, to prepare for the next consultation, etc. The time required depends on the care provider, the type of consultation that took place, and the patient. This inter-consultation time may be accounted for in preferred embodiments of the invention to more accurately determine the length of the timeslot to be reserved for a new healthcare appointment, by simply adding the inter-consultation time to the average duration for the appointment. This way, a more accurate reservation of time in the care provider's daily schedule takes place, further minimizing the delay of a care provider in his daily schedule when such inter-consultation time would not be reserved and/or further minimizing the advance/delay in the care provider's daily schedule compared to systems that account for a fixed, predetermined inter-consultation time.

In embodiments of the computer-implemented system for scheduling a new healthcare appointment according to the invention, as defined by claim 9, the second computer system is further configured for:
- receiving a cancellation for a scheduled healthcare appointment;
- identifying, upon receipt of the cancellation, a similar healthcare appointment of substantially equal duration with the same care provider, scheduled later than the healthcare appointment cancelled; and
- automatically generating an advance proposal for the patient of the similar healthcare appointment to advance the similar healthcare appointment to the timeslot of the healthcare appointment cancelled.

Thus, advanced embodiments of the system according to the invention also automatically re-schedule appointments with a care provider in case of late cancellation by a patient. Such late cancellations, similarly to no-shows, usually result in loss of time and income for the care provider. Instead of leaving an empty timeslot in the care provider's schedule, the system according to the invention may attempt to identify a later scheduled, similar healthcare appointment, for instance a healthcare appointment of substantially the same duration, for substantially the same reason or treatment, involving the same care provider or an associated care provider, etc. and then contacts the patient(s) of such similar healthcare appointment with a proposal to advance their healthcare appointment to the timeslot that became available as a result of the late cancellation. Again, such automated re-scheduling enhances the efficiency of the care provider and the satisfaction of patients who get the opportunity to advance their appointments and therefore reduce their waiting time for healthcare assistance.

In embodiments of the computer-implemented system for scheduling a new healthcare appointment according to the invention, as defined by claim 10, the second computer system is further configured for:
- distinguishing acute, chronical and check type of healthcare appointments; and
- automatically selecting in the appointment schedule an available timeslot of the average duration with a different care provider of a same or different healthcare cabinet in case of an acute healthcare appointment for which no timeslot of the average duration is available with the care provider within a day from receipt of the scheduling request for the new healthcare appointment.

Thus, the system according to the invention may be able to distinguish between acute, chronical and check type of healthcare appointments, for instance because the patient must give an indication thereof in the request for the new healthcare appointment or because the system can distinguish these three categories of appointments based on the consultancy type, reason or treatment. For acute healthcare appointments, advanced embodiments of the system shall automatically check the schedule of alternate care providers if there is no timeslot of appropriate duration available within the next 24 hours in the schedule of the preferred care provider. The system consequently shall propose the patient to schedule his acute appointment with an alternate care provider preferably associated with the same healthcare cabinet as the preferred care provider but alternatively associated with a different healthcare cabinet if no care provider of the same cabinet is available, such that the acute healthcare appointment can take place within the next 24 hours.

In embodiments of the computer-implemented system for scheduling a new healthcare appointment according to the invention, as defined by claim 11, the first computer system is further configured for:
- receiving location information from mobile user terminals of patients; and
- determining the start times and end times of past healthcare appointments also from said location information.

Indeed, the accuracy of the start times and end times of healthcare appointments, and consequently also of the duration of past healthcare appointments is increased in embodiments of the system according to the invention that considers location information of patients in addition to click events in electronic patient files. Such location information can for instance be obtained from smart devices carried by the patient and equipped with location technology like for instance a GPS (Global Positioning System) receiver or alternate location technologies. Such location information also may be exploited by the system according to the invention to determine travel times of patients to/from the healthcare cabinet or to determine travel times of the care provider in between home visit consultations. Knowledge of these travel times can be used to determine the length of time slots that must be reserved for future home visit appointments, or can be used for instance to determine appropriate times to send alerts or reminder messages to patients.

In addition to a computer-implemented system, the present invention also concerns a corresponding computer-implemented method for scheduling a new healthcare appointment comprising:
- registering respective times of click events in electronic patient files, an electronic patient file being a digital record comprising a patient identifier of a patient and health data concerning the patient;
- determining actual start times and end times of past healthcare appointments from the click events;
- maintaining an average duration for a healthcare appointment per care provider, per patient and per consultation type from the start times and the end times;
- receiving a scheduling request for the new healthcare appointment, the request comprising a patient identification, a care provider identification, and a consultation type identification; and
- determining an average duration for the new healthcare appointment based on the patient identification, the care provider identification and the consultation type identification; and
- selecting in an appointment schedule an available timeslot of the average duration for the new healthcare appointment.

According to a further aspect, the present invention concerns a controller as defined by claim 13, comprising at least one processor and at least one memory including computer program code, the at least one memory and computer program code configured to, with the at least one processor, cause the controller to perform the method of claim 12.

According to still another aspect, the present invention concerns a computer program product as defined by claim 14, comprising computer-executable instructions for performing the method of claim 12 when the program is run on a computer.

According to yet another aspect, the present invention concerns a computer readable storage medium as defined by claim 15, comprising computer-executable instructions for performing the method of claim 12 when the program is run on a computer.

### Brief Description of the Drawings

Fig. 1 illustrates an embodiment of the computer-implemented system 100 for scheduling healthcare appointments according to the invention;
Fig. 2 is a functional block scheme of the first computer system 101 in the embodiment of the computer-implemented system 100 for scheduling healthcare appointments of Fig. 1;
Fig. 3 is a functional block scheme of the second computer system 102 in the embodiment of the computer-implemented system 100 for scheduling healthcare appointments of Fig. 1; and
Fig. 4 shows an example embodiment of a suitable computing system 400 for implementing the first computer system 101 and/or second computer system 102 in embodiments of the computer-implemented system 100 for scheduling healthcare appointments according to the invention.

### Detailed Description of Embodiment(s)

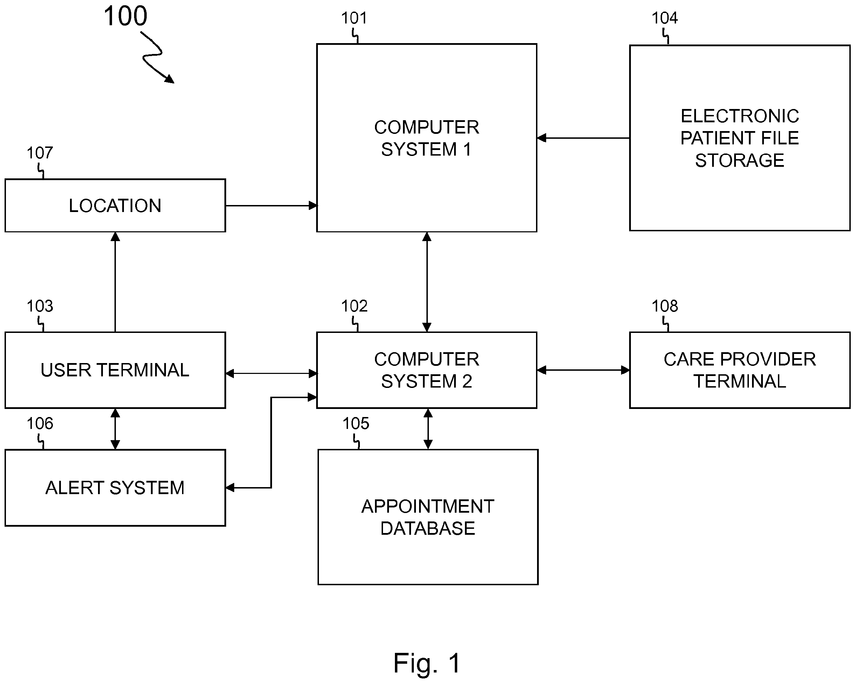

Fig. 1 shows the components of an example embodiment of the computer-implemented system 100 for scheduling healthcare appointments according to the invention. The system 100 comprises a first computer system 101 generally configured to monitor clicks in electronic patient files and to determine therefrom the average duration of a healthcare appointment per care provider, per patient and per consultation type. The first computer system 101 thereto is coupled with an electronic patient file store 104 memorizing electronic patient files, and with a location store 107 memorizing location information of user terminals. The electronic patient file store 104 and location store 107 may be integral part of the system 100 or may be external thereto. The system 100 further comprises a second computer system 102 generally configured to manage the scheduling of healthcare appointments in an appointment database 105 whereto it is coupled. The appointment database 105 also may be integral part of the system 100 or may be external thereto. The second computer system 102 further is coupled with user terminals 103, care provider terminals 108, and with an alert system 106 that communicates with the user terminals 103. The user terminals 103, care provider terminals 108 and alert system 106 may be integral part of the system 100 or they may be external thereto. In its most essential form, the system 100 according to the invention thus comprises only the first computer system 101 and second computer system 102, the second computer system 102 being equipped with a user interface terminal configured to receive requests for scheduling healthcare appointments from user terminals 103, whereas all other components drawn in Fig. 1 may be external to the system 100.

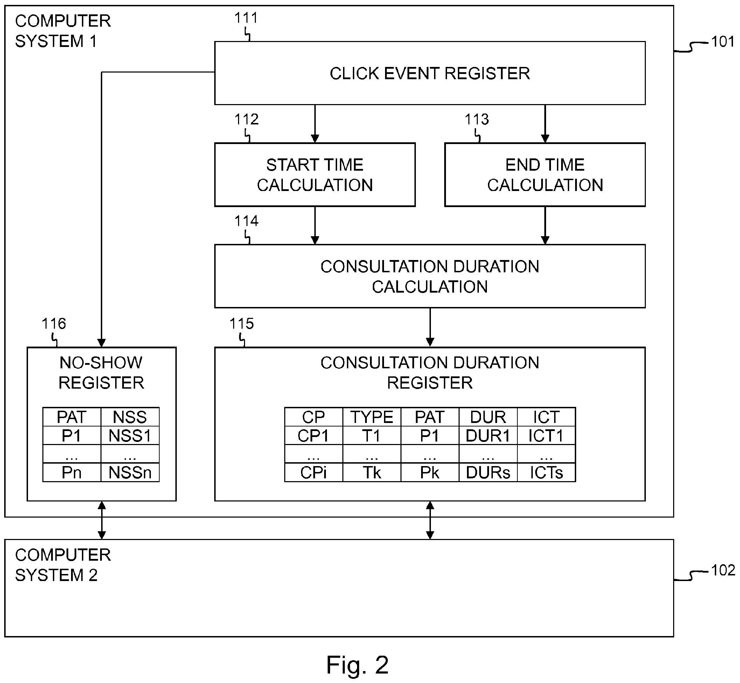

Fig. 2 shows the functional blocks that form part of the first computer system 101. A click event register 111 is configured to register the times of click events in electronic patient files that are stored in the electronic patient file store 104. A start time calculator 112 and end time calculator 113 use the time information available in the click event register 111 to respectively determine start times and end times of healthcare appointments, and feed these start times and end times to a consultation duration calculator 114 that determines the average duration of a consultation per care provider, per patient and per consultation type. The first computer system 101 further comprises a consultation duration register 115 wherein the average duration DUR1 ... DURs is stored per care provider CP1 ... CPi, per patient P1 ... Pk, and per consultation type T1 ... Tj. In addition to an average consultation duration DUR1 ... DURs, also an average inter-consultation time ICT1 ... ICTs may be stored per care provider, per patient and per consultation type in the consultation duration register 115. The optional determination and storage of the inter-consultation time ICT from information collected by the click event register 111 will be described in further detail below. The first computer system 101 further also comprises a no-show register 116 wherein a no-show score NSS1 ... NSSn is maintained per patient P1 ... Pn, and possibly also per care provider and per consultation type as it is expected that the no-show score may depend on the type of consultation (no-show behaviour may for instance be different for an urgent consultation or for a consultation involving a painful treatment) and possibly also on the care provider (the empathy of the care provider may for instance have a motivating effect on the patient, increasing his/her engagement). The optional determination of such no-show score NSS from information collected by the click event register 111 will be described in more detail below.

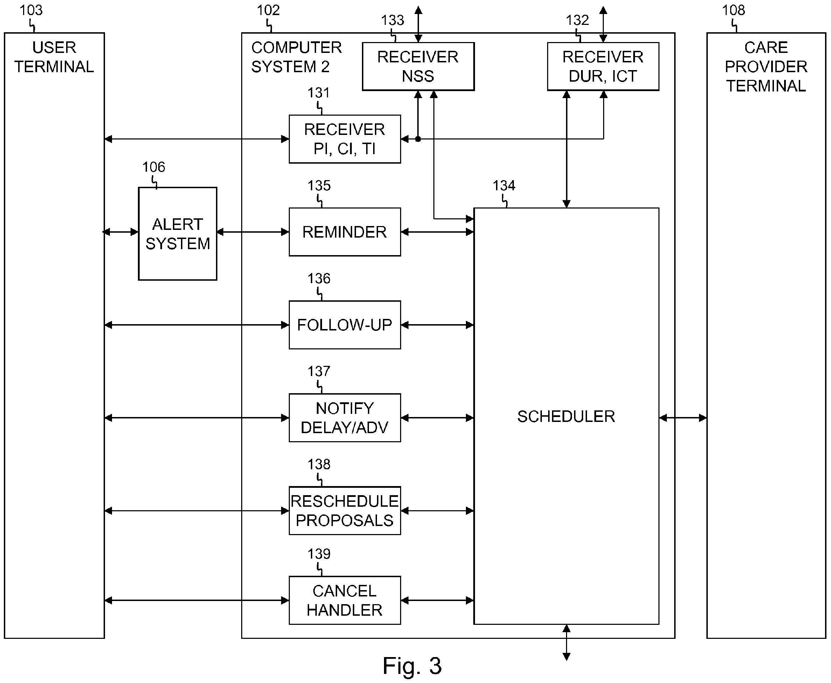

Fig. 3 shows the functional blocks that form part of the second computer system 102. The second computer system 102 comprises a user terminal interface 131 configured to receive the patient identifier PI, care provider identifier CI and consultation type identifier TI for a new healthcare appointment to be scheduled. The second computer system 102 further comprises an interface 132 towards the first computer system 101 configured to receive the average duration DUR and the inter-consultation time ICT for a given patient identifier PI, care provider identifier CI and consultation type identifier TI. The second computer system 102 also comprises an interface 133 towards the first computer system 101 configured to receive a no-show score NSS for a give patient identifier PI. The heart of the second computer system 102 is scheduling logic 134 that uses the information DUR, ICT, NSS received from the first computer system 101 to schedule healthcare appointments in an appointment database 105. The scheduler 134 further is configured to monitor progress in the daily schedule of healthcare appointments of one or plural care providers, and to interact with the users / patients in view of the progress. The second computer system 102 thereto comprises a reminder module 135, controlled by the scheduler 134 to remind a patient of upcoming healthcare appointments, a follow-up module 136 controlled by the scheduler 134 to interactively follow-up with the patient on a past healthcare appointment and/or prescribed therapy, a delay/advance notification module 137 controlled by the scheduler 134 to real-time notify a patient on delays and/or advances in a care provider's daily schedule, a reschedule proposal module 138 controlled by the scheduler 134 to generate reschedule proposals for one or several patients in case of late cancellation of a healthcare appointment by a patient, and a cancel handler module 139 controlled by the scheduler 134 to reschedule healthcare appointments of all patients in case of absence of a care provider.

The system 100 according to the invention represents a virtual back-office assistant for care providers that automates the management of a calendar for healthcare appointments. The most important functionality of this virtual back-office assistant, i.e. data collection through monitoring of click events in electronic patient files, dynamic real-time monitoring of progress in a daily schedule, automated rescheduling of appointments, and monitoring of no-show behaviour of patients will be described in detail in the following paragraphs.

### Data Collection

The click event register 111 in the first computer system 101 connects with the electronic patient file store 104 and monitors times whereon electronic patient files are opened and closed. The opening and closing times of electronic patient files enables the first computer system 101 to determine and maintain (i.e. create, modify, delete) the average duration DUR of a consultation per care provider CP, per patient PAT and per consultation type TYPE. The opening times of electronic patient files enable the start time calculation module 112 to determine the start times of consultations. The closing times of electronic patient files enable the end time calculation module to calculate the end times of consultations. The consultation duration calculation unit can determine the duration of a consultation by subtracting the start time of a consultation from the end time of a consultation. When multiple consultation durations have been calculated for the same care provider, patient and consultation type, an average consultation duration is determined by the consultation duration calculation unit 114 and stored in the consultation duration register 115. As is shown for instance in Fig. 2, the average duration of a consultation of type T1 by patient P1 with care provider CP1 is equal to DUR1. Similarly, the average duration of a consultation of type Tj by patient Pk with care provider CPp is equal to DURs. Knowledge of the average duration of a consultation per care provider, per patient and per consultation type, enables the second computer system 102 to select and reserve a free timeslot of appropriate length in the calendar of the care provider each time the patient uses the system to schedule a new consultation of a given type (urgency or treatment as indicated by the patient) with the care provider. In the second computer system 102, the receiver 131 receives a request for a healthcare appointment and extracts therefrom a patient identifier PI, a care provider identifier CI and a consultation type identifier TI. These three identifiers PI, CI and TI are used by receiver 132 to obtain the corresponding average duration DUR for the identified patient, care provider and type from the consultation duration register 115, and this average duration is used by the scheduler 134 to search one or more available timeslots in the appointment schedule of the identified care provider. The scheduler 134 may select one available timeslot, for example the earliest, and reserve that timeslot for the new healthcare appointment. Alternatively, if plural available timeslots were found, the scheduler 134 may inform the user on the available timeslots and give the user the possibility to choose the preferred timeslot for the new healthcare appointment. The scheduler 134 thereupon adapts the schedule of the care provider in the appointment database 105.

Embodiments of the first computer system 101 in addition to maintaining the average duration of consultations, may also register the time in between closing of an electronic patient file and opening of a following electronic patient file by the same care provider (or his assistant) in order to determine the average inter-consultation time ICT per care provider, per patient and per consultation type. This is indicated in Fig. 2 where the consultation duration register on top of the average duration DUR also registers the average inter-consultation time ICT per care provider, patient and consultation type. The inter-consultation time ICT is time used by the care provider for some smalltalk with the patient, to perform certain administrative tasks, and to clean his cabinet in order to prepare it for the next consultation. This inter-consultation time ICT depends on the care provider's attitude, the patient's attitude and the type of consultation, e.g. the treatment and medical tools involved. This average inter-consultation time interval can be accounted for by the scheduler 134 in the second computer system 102 when scheduling a new healthcare appointment. It will result in longer timeslots being searched for when a new appointment is scheduled, more accurately reflecting the time interval needed by the care provider for the consultation and shall consequently enable the care provider to minimize delay/advance with respect to his daily schedule.

In order to further improve the accurateness of the length of the timeslots to be reserved for healthcare appointments, the system 100 may further receive information on the location of patients and/or care providers, for instance obtain their GPS coordinates from a mobile client application running on their smartphones. Location information of the user terminals 103 may for instance be collected by a location store 107 that can be consulted by the first computer system 101 to further fine tune the duration of a consultation (the actual time spent by a patient in the medical cabinet of a care provider for a certain type of treatment) and/or to account for travel time of a patient when notifications are sent to the patient, and/or to account for travel time of the care provider in case of home visit consultations.

### Dynamic Real-Time Monitoring

The scheduling logic 134 in the second computer system 102 real-time monitors the delay/advance of a care provider in his daily schedule that is stored in the appointment database 105. Patients that have a consultation scheduled later that day, are automatically informed of the delay/advance as soon as the delay/advance exceeds a certain threshold such that these patients can show up later or earlier for their consultation. The scheduler 134 thereto instructs the delay/advance notification module 137 to inform the patients on the advance or delay. The delay/advance notification module 137 shall for instance send sms messages or Instant messages to the patients as soon as the delay/advance exceeds 30 minutes. The delay/advance notification module 137 may further be configured to adequately select the time whereon such notification is sent, for instance at least one hour in advance of the patient's appointment, or the notification time may be personalized accounting for the travel time of the patient, for instance one hour before the estimated time the patient will take off at home for the scheduled appointment. This way, both the care provider's waiting time and the patient's waiting time are minimized dynamically throughout the day, without disturbing the patient with numerous messages.

### Automated Re-scheduling

The scheduling logic 134 in the second computer system 102 automatically reschedules consultations with a care provider when the care provider indicates absence for a certain period, for instance in case the care provider is ill, attends a conference, etc. All patients that have a consultation scheduled within the period where the care provider will be absent, are contacted automatically by the cancellation handler module 139 with a notification indicating that their consultation is cancelled and in preferred embodiments of the system further automatically receive a proposed new date/time for their consultation that is selected by the scheduler 134 applying the above-described appointment scheduling algorithm.

The scheduling logic 134 also automatically attempts to re-schedule consultations in case of late cancellation of a consultation by a patient. Instead of leaving an empty timeslot in the care provider's calendar, the scheduler 134 shall identify similar consultations, i.e. timeslots of substantially the same duration, same type of treatment, same care provider, and instruct the reschedule proposal module 138 to contact the patients of such similar consultations with a proposal to advance their consultation to the timeslot that became available as a result of the late cancellation.

### Monitoring No-Show Behaviour

Embodiments of the first computer system 101 also automatically detect no-show behaviour of a patient and maintain a no-show score NSS per patient in a no-show register 116. The no-show score NSS1 ... NSSn of a patient P1 ... Pn is indicative for the risk that a patient will not show-up at a scheduled appointment. It is noticed that in more advanced embodiments no-show scores may be maintained per patient, per care provider and per consultation type. This no-show score may for instance be increased when each time the electronic patient file is not opened or when the electronic patient file is opened only for a limited time of for instance a few seconds. Such information can be obtained from the time information stored in the click event register 111. The no-show score of a patient may further be increased when other indicators of no-show behaviour are detected, for example when the patient ID is not scanned at the time a consultation was scheduled by that patient, when a healthcare appointment is rescheduled multiple times, when similar healthcare appointments are scheduled with multiple care providers, when the patient is slowly responding to an enquiry sent to the patient, when no confirmation is received after an appointment reminder is sent to the patient, etc. The latter no-show behaviour indicators can be detected by the second computer system 102, hence also the second computer system 102 may have the necessary interface to adapt the no-show scores in the no-show register 116, as is indicated by Fig. 2. The no-show behaviour score maintained for each patient is used to identify patients with high no-show risk, and therefore used by the scheduling logic 134 to determine if the reminder module 135 will send a reminder for a future consultation scheduled by the patient, how many reminders will be sent to that patient, and which communication channels (e-mail, sms, tel. call, Whatsapp, ...) will be used by the alert system 106 to remind the patient of the scheduled consultation. A cascade of non-intrusive alerting, like for instance Instant Messaging, up to intrusive notification, like for instance telephone calls, may be implemented depending on the no-show score of the patient. This way, the system 100 shall automatically attempt to increase patient engagement to healthcare appointments.

The no-show behaviour score of a patient also may be used as an indication of the patient engagement towards a therapy, medication, etc. The scheduling logic 134 therefore also may instruct the follow-up module 136 to automatically notify a patient or interact with a patient to follow-up on a prescribed therapy, use of medication, etc. in view of the patient's no-show behaviour score. In addition to increasing the patient's engagement, this enables to monitor effectiveness and side-effects of a therapy.

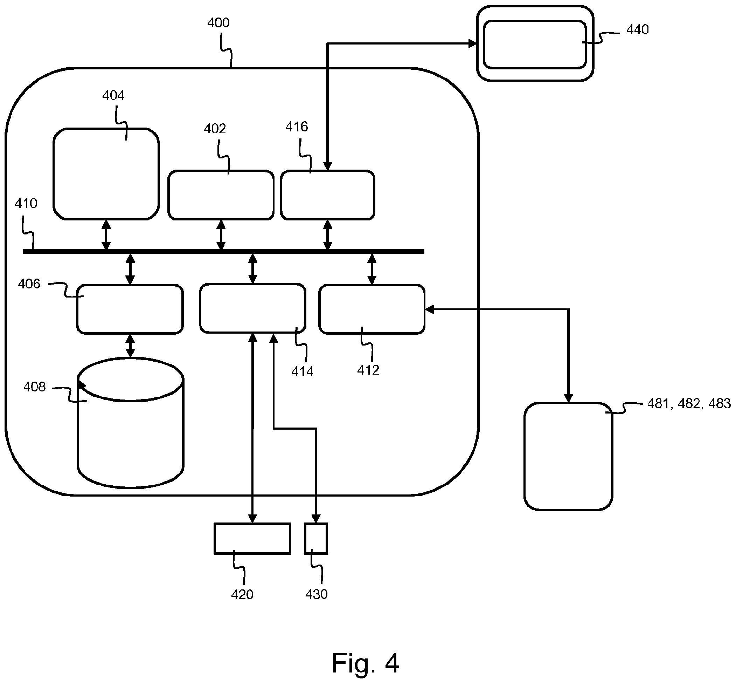

Fig. 4 shows a suitable computing system 400 enabling to implement embodiments of the first computing system 101 and/or the second computing system 102 in the above described embodiments of the system for scheduling healthcare appointments according to the invention. Computing system 400 may in general be formed as a suitable general-purpose computer and comprise a bus 410, a processor 402, a local memory 404, one or more optional input interfaces 414, one or more optional output interfaces 416, a communication interface 412, a storage element interface 406, and one or more storage elements 408. Bus 410 may comprise one or more conductors that permit communication among the components of the computing system 400. Processor 402 may include any type of conventional processor or microprocessor that interprets and executes programming instructions. Local memory 404 may include a random-access memory (RAM) or another type of dynamic storage device that stores information and instructions for execution by processor 402 and/or a read only memory (ROM) or another type of static storage device that stores static information and instructions for use by processor 402. Input interface 414 may comprise one or more conventional mechanisms that permit an operator or user to input information to the computing device 400, such as a keyboard 420, a mouse 430, a pen, voice recognition and/or biometric mechanisms, a camera, etc. Output interface 416 may comprise one or more conventional mechanisms that output information to the operator or user, such as a display 440, etc. Communication interface 412 may comprise any transceiver-like mechanism such as for example one or more Ethernet interfaces that enables computing system 400 to communicate with other devices and/or systems, for example with other computing devices 481, 482, 483. The communication interface 412 of computing system 400 may be connected to such another computing system by means of a local area network (LAN) or a wide area network (WAN) such as for example the internet. Storage element interface 406 may comprise a storage interface such as for example a Serial Advanced Technology Attachment (SATA) interface or a Small Computer System Interface (SCSI) for connecting bus 410 to one or more storage elements 408, such as one or more local disks, for example SATA disk drives, and control the reading and writing of data to and/or from these storage elements 408. Although the storage element(s) 408 above is/are described as a local disk, in general any other suitable computer-readable media such as a removable magnetic disk, optical storage media such as a CD or DVD, - ROM disk, solid state drives, flash memory cards, ... could be used. Computing system 400 could thus correspond to the first computer system 101 or the second computer system 102 in the embodiments illustrated by Fig. 1-3.

As used in this application, the term "circuitry" or "logic" may refer to one or more or all of the following:
(a) hardware-only circuit implementations such as implementations in only analog and/or digital circuitry and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and/or processor(s), such as microprocessor(s) or a portion of a microprocessor(s), that requires software (e.g. firmware) for operation, but the software may not be present when it is not needed for operation.

This definition of circuitry or logic applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented system (100) for scheduling a new healthcare appointment comprising:
- a first computer system (101) configured for:
- registering respective times of click events in electronic patient files, an electronic patient file being a digital record comprising a patient identifier of a patient and health data concerning said patient;
- determining actual start times and end times of past healthcare appointments from said click events; and
- maintaining an average duration for a healthcare appointment per care provider, per patient and per consultation type from said start times and said end times;
- a user interface terminal (103) configured for:
- receiving a scheduling request for said new healthcare appointment, said request comprising a patient identification, a care provider identification, and a consultation type identification; and
- a second computer system (102) configured for:
- receiving said patient identification, said care provider identification and said consultation type identification;
- receiving from said first computer system (101) an average duration based on said patient identification, said care provider identification and said consultation type identification; and
- selecting in an appointment schedule an available timeslot of said average duration for said new healthcare appointment.

2. A computer-implemented system (100) for scheduling a new healthcare appointment according to claim 1, wherein said first computer system (101) is further configured for:
- maintaining a no-show score per patient and increasing said no-show score whenever no electronic patient file is opened and/or no patient identification card is scanned for a scheduled healthcare appointment of said patient, and/or the electronic patient file is opened only during a time interval shorter than a certain time limit, and/or a new healthcare appointment is multiple times rescheduled by said patient, and/or similar new healthcare appointments are scheduled by said patient with multiple care providers, and/or the response delay to a request sent to said patient exceeds a certain threshold.

3. A computer-implemented system (100) for scheduling a new healthcare appointment according to claim 2, wherein said second computer system (102) is further configured for:
- determining if an automatic reminder is sent to a patient for a future healthcare appointment based on said no-show score of said patient.

4. A computer-implemented system (100) for scheduling a new healthcare appointment according to claim 3, wherein said second computer system (102) is further configured for:
- selecting a communication channel for said reminder based on said no-show score of said patient.

5. A computer-implemented system (100) for scheduling a new healthcare appointment according to claim 2, wherein said second computer system (102) is further configured for:
- following-up on a therapy or medication through automated interrogation of a patient after a healthcare appointment based on said no-show score of said patient.

6. A computer-implemented system (100) for scheduling a new healthcare appointment according to one of the preceding claims, wherein said second computer system (102) is further configured for:
- monitoring in said appointment schedule progression of scheduled appointments per care provider based on said start times and end times, and determining delay/advance of said care provider in view of his daily schedule;
- automatically notifying patients of one or plural upcoming appointments with said care provider on said delay/advance if said delay/advance exceeds a certain threshold; and
- registering an adequate delay for notifying patients of such delay/advance.

7. A computer-implemented system (100) for scheduling a new healthcare appointment according to one of the preceding claims, wherein said second computer system (102) is further configured for:
- automatically re-scheduling all appointments scheduled for a day with a care provider if said care provider indicates absence for said day; and
- generating re-schedule proposals for patients of said appointments, each re-schedule proposal comprising a respective new date and respective new timeslot of appropriate duration, available in said appointment schedule for said care provider.

8. A computer-implemented system (100) for scheduling a new healthcare appointment according to one of the preceding claims, wherein said first computer system (101) is further configured for:
- determining from said click events an inter-consultation time between closing of an electronic patient file and opening of a following electronic patient file; and
- maintaining an average inter-consultation time per care provider, per patient, and per consultation type therefrom,
and wherein said second computer system (102) is further configured for:
- receiving from said first computer system (101) an average inter-consultation time based on said patient identification, said care provider identification and said consultation type identification; and
- adding said inter-consultation time interval to said average duration when selecting in an appointment schedule an available timeslot for said new healthcare appointment.

9. A computer-implemented system (100) for scheduling a new healthcare appointment according to one of the preceding claims, wherein said second computer system (102) is further configured for:
- receiving a cancellation for a scheduled healthcare appointment;
- identifying, upon receipt of said cancellation, a similar healthcare appointment of substantially equal duration with the same care provider, scheduled later than said healthcare appointment cancelled; and
- automatically generating an advance proposal for the patient of said similar healthcare appointment to advance said similar healthcare appointment to the timeslot of said healthcare appointment cancelled.

10. A computer-implemented system (100) for scheduling a new healthcare appointment according to one of the preceding claims, wherein said second computer system (102) is further configured for:
- distinguishing acute, chronical and check type of healthcare appointments; and
- automatically selecting in said appointment schedule an available timeslot of said average duration with a different care provider of a same or different healthcare cabinet in case of an acute healthcare appointment for which no timeslot of said average duration is available with said care provider within a day from receipt of said scheduling request for said new healthcare appointment.

11. A computer-implemented system (100) for scheduling a new healthcare appointment according to one of the preceding claims, wherein said first computer system (101) is further configured for:
- receiving location information from mobile user terminals of patients; and
- determining said start times and end times of past healthcare appointments also from said location information.

12. A computer-implemented method for scheduling a new healthcare appointment comprising:
- registering respective times of click events in electronic patient files, an electronic patient file being a digital record comprising a patient identifier of a patient and health data concerning said patient;
- determining actual start times and end times of past healthcare appointments from said click events;
- maintaining an average duration for a healthcare appointment per care provider, per patient and per consultation type from said start times and said end times;
- receiving a scheduling request for said new healthcare appointment, said request comprising a patient identification, a care provider identification, and a consultation type identification; and
- determining an average duration for said new healthcare appointment based on said patient identification, said care provider identification and said consultation type identification; and
- selecting in an appointment schedule an available timeslot of said average duration for said new healthcare appointment.

13. A controller comprising at least one processor and at least one memory including computer program code, the at least one memory and computer program code configured to, with the at least one processor, cause the controller to perform the method of claim 12.

14. A computer program product comprising computer-executable instructions for performing the method of claim 12 when the program is run on a computer.

15. A computer readable storage medium comprising computer-executable instructions for performing the method of claim 12 when the program is run on a computer.
